# EUROPÄISCHE PATENTSCHRIFT

(11) **EP 1 154 739 B1**
(45) Veröffentlichungstag und Bekanntmachung des Hinweises auf die Patenterteilung: **31.07.2002**
(21) Anmeldenummer: 00906271.2
(22) Anmeldetag: 02.02.2000
(51) Int. Cl.: A61F 2/30

(54) **HOHLENDOPROTHESE**
HOLLOW ENDOPROSTHESIS
ENDOPROTHESE CREUSE

(30) Priorität: 16.02.1999 DE 19907489
(43) Veröffentlichungstag der Anmeldung: 21.11.2001
(73) Patentinhaber: ESKA Implants GmbH & Co., 23556 Lübeck (DE)
(72) Erfinder: GRUNDEI, Hans, D-23558 Lübeck (DE)
(74) Vertreter: Fuchs Mehler Weiss & Fritzsche
(86) Internationale Anmeldenummer: EP0000842
(87) Internationale Veröffentlichungsnummer: WO0048534

(56) Entgegenhaltungen:
- WO-A-98/26725
- DE-C- 4 106 971
- DE-C- 19 543 530
- FR-A- 2 744 010

## Beschreibung

Die vorliegende Erfindung betrifft eine Hohlendoprothese zum Einsatz in einem Röhrenknochen.

Endoprothesen zum Einsatz in einem Röhrenknochen sind in vielfältiger Ausgestaltung seit langem bekannt. Diese werden operativ nach Ausfräsung des Knochenmarkraumes in den Knochen gesetzt und dort entweder zementlos oder aber mit Knochenzement verankert.

Die vorliegende Erfindung betrifft eine Endoprothese der erstgenannten Klasse, d.h. eine zementlos in Röhrenknochen fixierbare Endoprothese. Auch aus dieser Klasse sind eine Fülle von unterschiedlichen Implantaten bekannt. Nur beispielhaft sei hier hingewiesen auf Implantate, die nach dem in der DE-A-41 06 971 beschriebenen Verfahren hergestellt sind. Weiterentwicklungen sind beispielsweise in der DE-A-195 43 530 beschrieben. Die erwähnten Implantate zeichnen sich dadurch aus, daß sie im wesentlichen einen massiven metallischen Grundkörper aufweisen, welcher zumindest teilweise bedeckt ist mit einer dreidimensionalen, offenmaschigen Raumnetzstruktur, in die hinein und durch die hindurch Knochenmaterial des das Implantat nach Implantation umgebenden Knochengewebes wächst, um so eine dauerhafte Sekundärfixation des Implantates im Röhrenknochen zu erzielen.

Dokument FR-A-2 744 010 offenbart eine Hohlendoprothese, die überwiegend aus einem metallischen Gitter und im übrigen Teil aus massivem Metall besteht.

Diese bekannten Implantate weisen erhebliche Vorteile gegenüber den mit Zement zu fixierenden Implantaten auf. So ist das Implantat nach vollständiger Einorganisierung an einer Vielzahl von Knochentrapekeln quasi freihängend in der umgebenden Spongiosa eingebettet, so daß es belastungsabhängige Ausgleichsbewegungen im Knochenmarksraum ausführen kann, wohingegen dies bei einem einzementierten Implantat praktisch nicht möglich ist, da dann diskrete Schichten (Knochen-Zement-Implantat) aufeinandertreffen und gegeneinander arbeiten. In diesem Fall liegt an der Schicht der Spongiosa die Knochenzementschicht, der sich das Implantat anschließt. Ausgleichsbewegungen unter Belastungen führen zu einer Lockerung des Knochenzementes und damit zu einer Lockerung des Sitzes des Implantates im Knochen.

Aber auch bei den beschriebenen zementlos zu fixierenden Implantaten kann es aufgrund der Massivität des Grundkörpers zu Problemen kommen aufgrund des sehr unterschiedlichen E-Modüls des Implantates einerseits und des Knochen andererseits. So ist der E-Modul des Implantates erheblich höher als jener des Knochens, was trotz der günstigen freien Aufhängung in der Spongiosa zu Problemen hinsichtlich einer Lockerung des Implantates führen kann.

Vor diesem Hintergrund ist es die Aufgabe der vorliegenden Erfindung, ein Implantat vorzuschlagen, welches eine weitaus geringere Tendenz hat, in seinem Sitz im Röhrenknochen gelockert zu werden.

Gelöst wird diese Aufgabe allgemein durch eine Hohlendoprothese, die überwiegend aus einem metallischen Gitternetzwerk besteht, das zumindest an seinen Knotenpunkten mit metallischen Partikeln bestückt ist, die gebildet sind aus einem Grundkörper und wenigstens drei von diesem radial abragenden Zapfen und die integrale Bestandteile vom das Gitternetzwerk bildendem Material sind, und die im übrigen Teil aus massivem Metall besteht.

Die Ausbildung als Hohlimplantat durch das Gitternetzwerk bewirkt eine bedeutende Angleichung der E-Moduln der Endoprothese und des Knochens.

Mit anderen Worten wird dem Implantat trotz seiner tragenden Struktur eine gewisse Elastizität verliehen, die es ihm gestattet, in etwa knochenähnlicher Weise auf die auftretenden Belastungen zu reagieren.

Für den Wachstumsanreiz des die Endoprothese umgebenden Knochenmaterials dienen die metallischen Partikel, welche der Endoprothese ein aggressives Äußeres verleihen, wodurch das umgebende Knochenmaterial zur Blutung und damit zum Aufbau und Einorganisieren von Knochentrapekeln in die Strukturen hinein angeregt wird.

Damit gleichwohl eine hinreichende Stabilität der Endoprothese erhalten bleibt, ist vorgesehen, daß ein Teil weiterhin aus massivem Metall besteht, jedoch zu einem geringeren Teil als aus dem Gitternetzwerk. Dieser übrige massive Teil nimmt eine Führungsfunktion der Endoprothese wahr, und zwar in jenem Bereich, in dem nur geringere Kräfte von der Endoprothese in das Knochenmaterial hineingeleitet werden muß. Im Falle der Ausbildung der erfindungsgemäßen Hohlendoprothese als Hüftstiel eines künstlichen Hüftgelenks bildet der massive Teil aus Metall das distale Ende des Hüftstiels.

Besonders bevorzugt wird, wenn diese zu wenigstens 60% aus dem metallischen Gitternetzwerk besteht. Dementsprechend werden die restlichen 40% massiv ausgebildet, und zwar im Falle eines Hüftstieles am distalen Teil und zum geringeren Anteil im proximalen Bereich zur Ankoppelung einer künstlichen Gelenkpfanne. Diese Endoprothese hat demnach mindestens einen 60%igen Bereich, bei dem der entsprechende E-Modul jenem des Knochens angenähert ist.

Zur Erhöhung der mechanischen Stabilität unter nur geringer Erhöhung des E-Moduls des aus Gitternetzwerk bestehenden Abschnittes der Endoprothese kann es vorteilhaft vorgesehen sein, daß mit dem Gitternetzwerk Versteifungsstreben ausgebildet sind. Im Falle beispielsweise einer Hüftstielendprothese würden diese parallel von proximal nach distal hin zu dem massiven metallischen distalen Abschnitt verlaufen.

Zusammenfassend wird nochmals betont, daß die Ausbildung eines Großteils der Endoprothese als metallisches Gitternetzwerk im Hinblick auf die Angleichung der E-Moduln wesentlich ist und die Bestückung mit den beschriebenen Partikeln wesentlich ist im Hinblick auf den Wachstumsanreiz des die Endoprothese umgebenden Knochenmaterials zur raschen und dauerhaften Einorganisierung in die und durch die Ebene der Oberflächenstruktur hindurch, die von den Partikeln gebildet wird, und sodann in das Innere der Endoprothese hinein.

Die Erfindung wird anhand zweier Ausführungsbeispiele näher erläutert. Hierbei zeigt:
- Figur 1: eine Hohlendoprothese in Ausbildung eines Hüftstieles eines künstlichen Hüftgelenkes mit vergrößert dargestelltem Partikel an der Außenseite des Gitternetzwerkes, und
- Figur 2: eine ähnliche Ansicht eines Hüftstiels wie in Figur 1 gemäß einer abgewandelten Ausgestaltung, und
- Figur 3: eine Schnittansicht entlang der Linie III-III in Figur 2.

Nachfolgend sind entsprechende Teile mit denselben Bezugszeichen versehen.

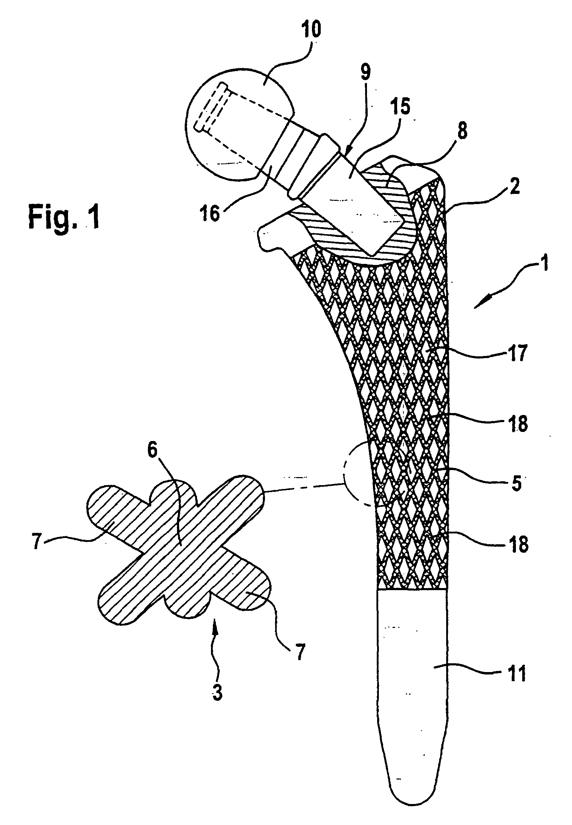

Figur 1 zeigt in teilweisem Schnitt einen Hüftstiel 1, also einen Femurteil eines künstlichen Hüftgelenkes.

Der Hüftstiel 1 weist einen künstlichen Gelenkkopf 10 des Hüftgelenkes auf, der mittels eines Doppelkonusadapters 9 mit Steckkonen 15 und 16 mit dem massiven Anschlußteil 8 des Hüftstiels 1 mit ausgebildeter Steckhülse verbunden ist.

Zum überwiegenden Teil besteht der Hüftstiel 1 aus einem metallischen Gitternetzwerk 2 mit offenen Maschen 17 zwischen denen das Gitternetzwerk 2 bildenden Stegen 18.

Zumindest an den Knotenpunkten 5, an denen sich die Stege 18 kreuzen, sind metallische Partikel 3 vorgesehen. Diese sind integral mit dem Gitternetzwerk 2 ausgebildet, vorzugsweise in einem feingießtechnischen Wachsausschmelzverfahren mit dem Gitternetzwerk gegossen worden.

Die abschnittsweise Vergrößerung zeigt das Partikel 3 deutlicher. Dieses besteht aus einem Grundkörper 6 und vorliegend fünf radial davon abragenden Zapfen 7. Diese Ausbildung schafft eine enorme Aggressivität der Oberfläche der Hohlendoprothese gegenüber dem sie umgebenden Knochenmaterial nach Implantation im Femur. Diese mechanische Aggressivität fördert maßgeblich das Wachstum der Knochentrapekel.

Der distale Teil des Hüftstiels 1 ist vorliegend als massiver Teil 11 ausgebildet, der eine Führungsfunktion für die Endoprothese ausübt. Diese Führungsfunktion führt zu einer Zentrierung des Hüftstieis im Knochenmarksraum des Femurs.

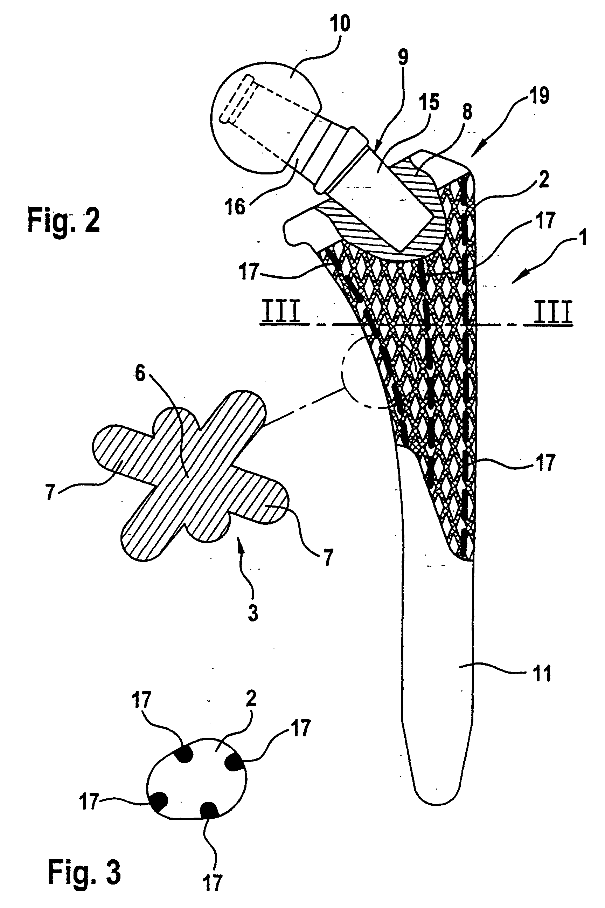

Die in Figur 2 dargestellte Hohlendoprothese unterscheidet sich in hauptsächlich zwei Merkmalen von jener in Figur 1. Daher wird nachfolgend vorwiegend auf diese Unterschiedsmerkmale eingegangen:

Bei der dargestellten Ausführungsförm sind Versteifungsstreben 17 vorgesehen, die vom proximalen Ende 19 des Hüftstiels 1 hin zum distalen massiven Teil 11 verlaufen. Ihre Anordnung auf dem Gitternetzwerk 2 ist anschaulich in Figur 3 dargestellt, die einen schematischen Schnitt entlang der Linie III-III in Figur 2 darstellen. Erkennbar ist der angedeutete Verlauf je einer Versteifungsstrebe 17 im medialen, lateralen, dorsalen und ventralen Bereich des Hüftstiels 1. Hierdurch wird eine aufbaubedingte etwaig zu geringe Stabilität des Gitternetzwerks 2 kompensiert, wobei jedoch der E-Modul weiterhin dem E-Modul von Knochen angenähert bleibt.

Insgesamt ergibt sich ein schnelles Einwachsverhalten der Hohlendoprothese aufgrund der enormen mechanischen Aggressivität ihrer Außenstruktur im Bereich des Gitternetzwerks 2 und eine hohe Langzeitstabilität aufgrund der Ausbildung des überwiegenden Teils als Gitternetzwerk 2 wegen der dann angenäherten Werte der E-Moduln der Endoprothese und des Knochens.

## Patentansprüche

1. Hohlendoprothese, die überwiegend aus einem metallischen Gittemetzwerk (2), das zumindest an seinen Knotenpunkten (5) mit metallischen Partikeln (3) bestückt ist, die gebildet sind aus einem Grundkörper (6) und wenigstens drei von diesem radial abragenden Zapfen (7) und die integraler Bestandteil vom das Gitternetzwerk (2) bildenden Material sind, und im übrigen Teil (11) aus massivem Metall besteht.

2. Hohlendoprothese nach Anspruch 1, die zu wenigstens 60% aus dem metallischen Gitternetzwerk (2) besteht.

3. Hohlendoprothese nach Anspruch 1 oder 2 als Hüftstielendoprothese, bei der das distale Ende des Hüftstiels der massive Teil (11) aus Metall ist.

4. Hohlendoprothese nach einem der Ansprüche 1 bis 3, bei der einstückig mit dem Gitternetzwerk (2) Versteifungsstreben (17) ausgebildet sind.

5. Hohlendoprothese nach Anspruch 3 und 4, bei der die Versteifungsstreben (17) vom proximalen Ende (19) hin zum distalen massiven Teil (11) verlaufen.

## Claims

1. Hollow endoprosthesis which consists predominantly of a metallic lattice network (2), which is fitted at least at its intersections (5) with metallic particles (3), which are formed from a base body (6) and at least three pins (7) projecting radially from the latter and the integral components are of the material forming the lattice network (2), and otherwise part (11) consists of solid metal.

2. Hollow endoprosthesis according to claim 1, at least 60% of which consists of the metallic lattice network (2).

3. Hollow endoprosthesis according to claim 1 or 2 as hip stem endoprosthesis, in which the distal end of the hip stem of the solid part (11) is made from metal.

4. Hollow endoprosthesis according to one of claims 1 to 3, in which stiffening supports (17) are designed to be integral with the lattice network (2).

5. Hollow endoprosthesis according to claim 3 and 4, in which the stiffening supports (17) run from the proximal end (19) to the distal solid part (11).

## Revendications

1. Endoprothèse creuse composée principalement d'un réseau de grille métallique (2) équipé au moins en ces points nodaux (5) de particules métalliques (3) formées d'un corps de base (6) et d'au moins trois chevilles (7) faisant saillie en rayon depuis ce corps et étant parties intégrantes du matériau formant le réseau de grille (2) et pour la partie restante (11) composée de métal solide.

2. Endoprothèse creuse selon la revendication 1 composée au moins à 60% du réseau de grille métallique (2).

3. Endoprothèse creuse selon la revendication 1 ou 2 comme endoprothèse de tige de la hanche, dans laquelle l'extrémité distale de la tige de hanche est la partie solide (11) en métal.

4. Endoprothèse creuse selon l'une des revendications 1 à 3, dans laquelle des supports de renforcement (17) sont formés d'une pièce avec le réseau de grille (2).

5. Endoprothèse creuse selon les revendications 3 et 4, dans laquelle les supports de renforcement (17) vont de l'extrémité proximale (19) jusqu'à la partie distale solide (11).
